Europäisches Patentamt

European Patent Office

Office européen des brevets

(19)

(11) Publication number : **0 009 845**
**B2**

(12) # NEW EUROPEAN PATENT SPECIFICATION

(45) Date of publication of the new patent specification :
**11.11.87**

(51) Int. Cl.⁴ : **A 61 K 31/43**, A 61 K 9/00

(21) Application number : **79200545.6**

(22) Date of filing : **27.09.79**

(54) Process for the manufacture of sodium amoxicillin preparations.

(30) Priority : **03.10.78 NL 7809986**
**03.01.79 NL 7900021**

(43) Date of publication of application :
**16.04.80 Bulletin 80/08**

(45) Publication of the grant of the patent :
**27.10.82 Bulletin 82/43**

(45) Mention of the opposition decision :
**11.11.87 Bulletin 87/46**

(84) Designated contracting states :
**AT CH DE GB IT NL SE**

(56) References cited :
**DE-A- 2 623 835**
**GB-A- 1 463 563**
**US-A- 3 969 524**
**CRC Handbook of Chemistry and Physics, 60th Edition, P.D-149**
**The file contains technical information submitted after the application was filed and not included in this specification**

(73) Proprietor : **Gist - Brocades N.V.**
**Wateringseweg 1 P.O. Box 1**
**NL-2600 MA Delft (NL)**

(72) Inventor : **De Jonge, Hayo**
**Fresialaan 27**
**NL-2106 BR Heemstede (NL)**
Inventor : **Groenendaal, Jan Willem**
**Robert Kochlaan 434**
**NL-2035 BP Haarlem (NL)**
Inventor : **Sijbrands, Gerrit Jan**
**Piet Leffertsstraat 8**
**NL-2042 EH Zandvoort (NL)**
Inventor : **Bagerman-Deetman, Annita**
**Oudenhove 116**
**NL-4901 CC Oosterhout (NL)**

(74) Representative : **Van der Straaten, Jan Anthony et al**
**c/o GIST-BROCADES N.V. Patents and Trademarks Department Martinus Nijhofflaan 2 P.O. Box 1**
**NL-2600 MA Delft (NL)**

## Description

The invention relates to a process for the preparation of a sodium amoxicillin preparation of improved quality, suitable for the use in injection preparations, and more particularly to a process for the preparation of sodium amoxicillin of improved quality by a freeze drying process.

Such a process is known from e. g. the Dutch published patent application no. 7 707 494. This patent application discloses a process consisting of freeze drying a solution of sodium amoxicillin a solvent system containing water and as a stabilizer at least a secondary or tertiary alkanol with 4 or 5 carbon atoms, which is soluble in water at 25 °C for at least 5 % by weight, while preferably 4-50 % by weight of the secondary or tertiary alkanol is present.

The tertiary alkanol is preferably t-butanol, but the solvent system may also contain small amounts of other pharmaceutically acceptable solvents, such as primary alcohols.

A disadvantage of these preparations is the presence of solvent residues, which, although they are toxicologically acceptable, are foreign to the animal or human body and consequently may give rise to several undesired side reactions after the administration of the injection preparation. The dry product may contain up to 6 % of these solvent residues.

It was also known to prepare crystalline cephalosporins for parenteral administration by freeze drying. For example, the US patent no. 4 029 655 and more especially the Belgian patent no. 861 135, relate to the preparation of stable sodium cephalotin powder for parenteral administration by means of freeze drying a solution of sodium cephalotin in an aqueous solution containing 2-10 % alcohol or acetone. Further, the Dutch published patent aplication no. 7 712 823 may be mentioned, which relates to the preparation of crystalline, easily dissolving sodium cephazolin by means of freeze drying an aqueous solution containing 2-25 % of alkanol, while cooling the starting solution slowly. However, the same disadvantages as mentioned above hold for the preparations thus obtained.

On the other hand, the patent literature of recent years shows that much activity is being directed to the search for amoxicillin preparations, which easily may be converted into sufficiently stable injection solutions and which are sufficiently stable in the dry form as well. It emerges from this patent literature, that the general developed conception of people skilled in the art is, that for the preparation of injectable preparations of amoxicillin, the usual methods, as applied for earlier known semi synthetic penicillins such as ampicillin, certainly cannot be used without any additional measures, as a consequence of the clearly differing chemical and physical properties of amoxicillin.

Therefore, on the one hand all sorts of proposals are made in order to come to adapted compositions, containing the usual alkali metal salts of amoxicillin and derivatives thereof and on the other hand the people skilled in the art seem to incline to search for readily constitutable and well injectable preparations, containing amoxicillin salts with alternative cations.

For example, the Dutch published patent application no. 7 509 701 describes a process for the preparation of the choline salt and the N-methyl-D-glucamine salt of amoxicillin respectively according to methods known per se, while in the Dutch published patent application no. 7 509 698 the preparation of the arginin salt of amoxicillin is disclosed. These salts of amoxicillin should lead to novel, non-toxic, parenterally administrable forms of amoxicillin, under preservation of the antibiotic properties. Moreover, on page 1 it is explicitly stated, that amoxicillin itself as well as its salts which are known up to now, cannot be administered parenterally in a satisfactory way. Also in example 4 of the Japanese published patent application 51 032 723, the preparation is disclosed of a suitable injectable solution, containing amoxicillin and the sodium salt of glycine.

In the German published patent application no. 2 540 523 a process is described for the preparation of salts of D-α-carboxyamino-p-hydroxybenzylpenicillin, intended for the preparation of satisfactorily injectable amoxicillin preparations. But also on page 2 of this application it is again explicitly stated, that the preparation of injectable amoxicillin preparations was found to be much more difficult than was initially expected by skilled people, which difficulties were attributed to the instability of amoxicillin solutions, caused by the decomposition of amoxicillin salts in aqueous solutions.

According to this last mentioned patent application, preferably mixtures of the sodium salt of amoxicillin and the disodium salt of D-α-carboxyamino-p-hydroxybenzyl penicillin should be applied.

In the Dutch published patent application no. 7 602 180 a process is described for the preparation of an injectable preparation of amoxicillin, which possesses a good stability and is well tolerated at administration. This preparation comprises a powder which may easily be converted into an injectable preparation by addition of an aqueous vehiculum. The powder consists of fine particles of amoxicillin trihydrate, coated with a dispersing agent, the ratio between amoxicillin trihydrate and dispersing agent being of from 1.000 : 1 to 20 : 1. The fine particles under consideration should have a diameter of from 2 μ to 20 μ in average, while at least 95 % should have a diameter between 0.5 μ and 50 μ, while from 10 up to 100 % of the surface should have been coated with dispersing agent. A proposed agent is a mixture, containing at least one polymeric material soluble in water and having an average molecular weight of from 6.000 to 40.000, such as polyvinylpyrrolidone, vinylpyrrolidone/vinylacetate copolymers, sodiumcarboxymethylcellulose, polyvinylalcohol, dextran and sodiumalginate and preferably polyvinylpyrrolidone, and a wetting agent such as lecithine, a phospholipide, sorbitan fatty acid esters and more particularly

2

lecithine.

For similar reasons research has been directed to amoxicillin derivatives, which yield amoxicillin on decomposition in the body. This principle is illustrated by the disclosure of US patent no. 4 035 381 and the Dutch patent application no. 7 701 480.

As a result of research and experimentation it was now surprisingly found, that improved dry sodium amoxicillin preparations, having a relatively long stability and which easily may be converted into stable, well injectable solutions can be prepared by adding gradually, but as fast as possible and with thorough mixing an excess of sodium hydroxide from 9 to 15 mol% over that required to form amoxicillin to an aqueous suspension of amoxicillin trihydrate until the amoxicillin has completely dissolved, subsequently neutralizing immediately a part of the excess of the base with hydrochloric acid at a temperature between 0° and + 30 °C to a final excess of sodium hydroxide from 3 to 5 mol% and submitting the solution obtained successively to sterile filtration, freezing and freeze drying. The partial neutralization is preferably carried out at a temperature between 20 and 25 °C.

Preferably the starting amounts of amoxicillin trihydrate, sodium hydroxide and hydrochloric acid are selected such, that a final concentration of 2.5-15 % by weight of sodium amoxicillin and more preferably of less than 5 % by weight is reached in the solution to be frozen.

When preparing the clear solution of the amoxicillin salt, high local base concentrations should be avoided. It is therefore advantageous to add an aqueous solution of sodium hydroxide gradually and under vigorous stirring to the suspension of amoxicillin trihydrate and to neutralize a part of the excess sodium hydroxide as fast as possible. As a result of partial neutralization about 10 mol% of sodium chloride is present in the solution.

The frozen solution may be freeze dried in bulk or in injection flasks.

It will be appreciated that filtration, freezing and freeze drying have to be carried out under asceptical conditions.

If the solution is frozen and freeze dried in flasks, preferably injection flasks and rubber stoppers especially designed for freeze drying are used. The flasks are filled in such a way, that they contain an amount of 0.10-5 g and preferably 0.25, 0.5 and 1 g of the dry final sodium amoxicillin preparation.

The freezing and freeze drying may be carried out by the application of equipment which is generally used for the purposes, such as a Sec. Froid SA CH-1024 lyolab D laboratory freeze drying equipment or a Leybold production freeze drying equipment. It will be appreciated that the freezing may also take place outside the actual freeze drying equipment.

The amoxicillin solution is preferably cooled in 0.5 to 2.5 hours to — 20 °C to — 70 °C and more preferably in 0.5 to 1.0 hour to — 20 °C to — 30 °C at atmospheric pressure. Subsequently the pressure is reduced to 9.0-12.0 N/M$^2$ and the temperature is slowly raised to 0 °C in 24-30 hours and preferably in about 25 hours, while the vacuum slowly rises to about 8 N/M$^2$. The temperature is then raised by heating to ambient temperature in 4-5 hours while maintaining the vacuum at 8 N/M$^2$.

When freeze drying in bulk is applied, the amoxicillin solution is suitably poured under aseptical conditions in stainless steel trays or plates of sterilized plastic foil supported by a sterilized metal frame having a vertical rim of a height up to about 4 cm.

The plastic foil has to be secured in such a way, that an optimal contact is obtained of the solution with the drying plates. The final level of the liquid may be 0.5 to 3 cm and is preferably about 1 cm. The drying plates of the freeze drying equipment are preferably previously cooled to a temperature of from — 25 °C to — 50 °C and preferably to about — 40 °C.

The cooling capacity of the freeze drying equipment has to be large enough to freeze the solution within 60 to 30 minutes and preferably within 50 to 40 minutes at a temperature of from — 10 °C to — 30 °C and preferably of from — 15 °C to — 20 °C. The time between the suspension of the amoxicillin trihydrate and the closing of the freeze drying equipment after bringing in the flasks or pouring out the solution on the plates preferably does not amount to more than 30 minutes, while the time which is necessary for the steps from the preparation of the solution up to and including the freezing, preferably should not exceed 90 minutes.

When freeze drying in bulk is applied, it is suitable to switch on the condensor and to lower the pressure in the drying room to about 100 N/M$^2$ as soon as the formation of ice occurs and the temperature of the product has been lowered to about — 10 °C.

After 0.5-2 hours at the same pressure, the cooling of the plates is switched off and the heater of the drying plates is switched on, while the temperature of the heating liquid is raising slowly and linearly within 60 minutes to room temperature. The subsequent drying period is 20-40 hours and preferably about 30 hours. During the whole freezing and freeze drying process, the temperatures of the product, the heating liquid of the plates, the drying plates and the condenser and the pressure in the drying room and in the condenser are recorded.

The secondary drying may, for instance, take place during 5-8 hours at a temperature of 30 °C of the drying plates. The vacuum of the drying room is eliminated by the supply of dry nitrogen, which has been filtered through a 0.2 μm filter, after which the dried sodium amoxicillin is removed from the drying room and is stored under nitrogen at a temperature of 0-10 °C under aseptical conditions.

Finally, the sodium amoxicillin is ground under aseptical conditions to a particle size, at which a sieve of 2 mm may be passed, and then amounts of 250, 500 or 1.000 mg are filled into sterilized injection flasks under dry and aseptical conditions.

When the sodium amoxicillin solution is frozen and freeze dried in flasks, flasks containing 0.5 or 1.0 g of the dried product may be prepared by starting from 10 or 20 ml of a 5 % by weight of sodium amoxicillin solution.

Surprisingly it was found that the process of the invention results in a dry sodium amoxicillin preparation which contains only 4.5-3.0 % and usually about 3.5 % by weight of decomposition products and which shows an improved stability as regards further decomposition of the product.

Solutions prepared from the dry preparation also show an improved stability. It is a further advantage of the product that it does not contain stabilizers or organic solvents or other chemicals that may cause undesired side-effects, such as allergic reactions.

In the dry preparation, 5-12 mg and preferably 7-8 mg of sodium chloride are present per 500 mg of sodium amoxicillin.

It has to be admitted that it was known to prepare sodium ampicillin by means of freeze drying. For example, according to the German patent application 2 623 835 an amount of sodium hydroxide, sodium bicarbonate or sodium carbonate ranging from 90 to 100 mol% with respect to the amount of ampicillin is added in aqueous solution to an aqueous suspension of ampicillin at a maximum temperature of 4 °C in such a way that too high local base concentrations are entirely absent. The solution obtained is then immediately submitted to sterile filtration, freezing and lyofilisation.

However, in view of the well known differences in chemical and physical properties between ampicillin and amoxicillin, it was certainly not predictable to people skilled in the art, that a useful method for the preparation of sodium ampicillin could be adapted for the preparation substantially pure sodium amoxicillin. Where so much efforts have failed already or have led to only partial results, it is virtually impossible to predict the suitability of a novel method.

In view of the teachings of German patent application 2 623 835 and of the conceptions of the people skilled in the art of penicillin chemistry, as reflected by the prior art discussed above, it was not obvious to use an excess of sodium hydroxide. According to the German specification an excess of base should be prevented.

According to Canadian Journal of Pharmaceutical Sciences 12 (1977) no. 3, p. 83, the right column, the maximum stability of amoxicillin is at pH 5.77 and the rate of decomposition increases only when the pH is lowered to 5.5 or raised to 6.5, while the optimum stability of aqueous amoxicillin solutions requires a citrate buffer with a pH between 5.8 and 6.5. These statements clearly lead away from the process of the invention, in which a pH of 8-9 is reached. Mixtures of amoxicillin trihydrate and sodium carbonate from which on addition of water sodium amoxicillin is formed, are described in US patent 3 969 524.

The preparations obtained according to the present invention may be converted to the desired aqueous injection solutions according the methods known per se.

The invention is illustrated by the following examples.

## Example 1

64.5 mmoles (27.06 g) of amoxicillin trihydrate are suspended by means of an lla homogenizer of the type X-1020 at a temperature of 20-25 °C within 5 minutes and in about 350 ml of sterile pyrogenfree water. 74.2 mmoles (2.97 g) of sodium hydroxide in 50 ml of sterile pyrogenfree water are added dropwise under vigorously stirring within 5 minutes. The clear solutions is immediately neutralized with 6.45 mmoles (12.9 ml of 0.50 N) hydrochloric acid. The solution is transmitted into a measuring-flask of 500 ml and sterile pyrogenfree water is added for adjustment of the exact volume. The solution is filtered bacteria-free under aseptical conditions by means of for example stainless steel Sartorius filter equipment of the type 16245 provided with a Sartorius membrane filter having a pore size of 0.2 μm of the type 11307 connected to a Sartorius aircompressor of the type Al-17.10 ml portions of the filtered solution are filled into 20 ml injection flasks by means of a Brand dispensor of the type 7050-15 under aseptical conditions. Temperature sensors belonging to the Sec. Froid freeze drying equipment of the type Lyolab D are fixed in three flasks. The flasks are provided with rubber stoppers which are especially designed for freeze drying. The filled flasks are placed on one of the three drying plates of the dryingroom after the plates have been adjusted to a temperature of — 20 to — 25 °c by means of the freezing-bath, whereby the dryingroom is flushed with about 600 l/hour of nitrogen gas, filtered through 0.2 μm filter. A temperature sensor is connected in the drying plate whereon the flasks are placed. During the whole process thereafter, the temperature of the three flasks and of the drying plate is recorded by means of a Sec. Froid recorder.

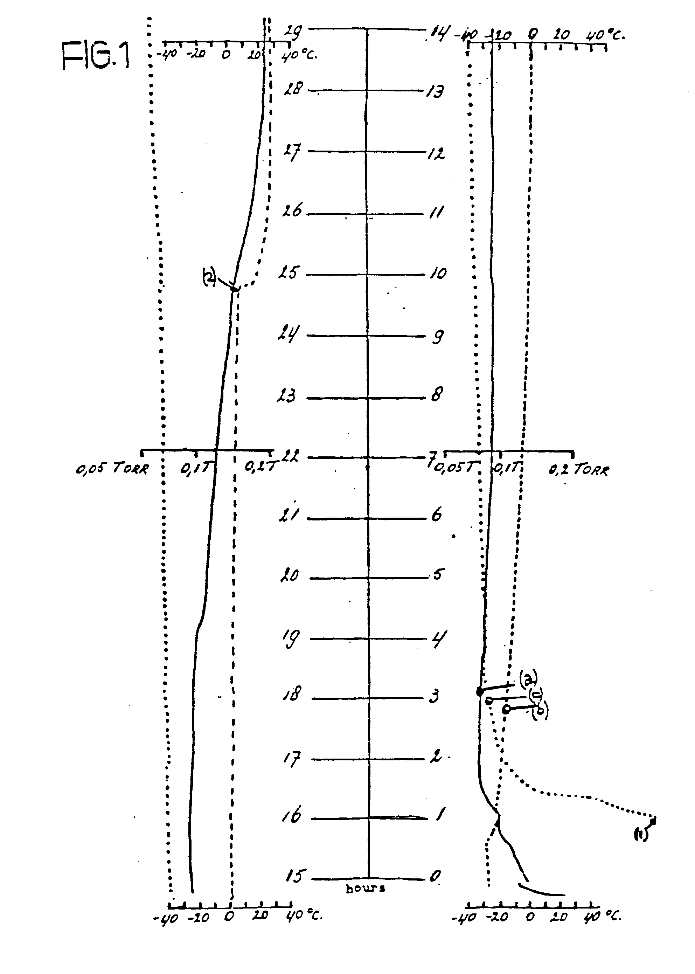

In figure 1 the course of respectively the temperature of the product, the temperature of the drying plate and the absolute pressure with the time, are indicated from the start of the freezing by the way of a characteristic example of the present process. The course of the temperature of the product is indicated by means of curve (a), the course of the temperature of the drying plate by means of curve (b) and the course of the absolute pressure by means of curve (c).

The point (1) in curve (c) indicates the switch on of the vacuum pump, while the point (2) in curve (b) indicated the switch on of the heating of the drying plate.

After the formation of ice in the flasks has occurred and the temperature has decreased within 1 hour

4

to — 20 °C, the dryingroom is evacuated after the nitrogen supply has been cut off and the condenser is switched on.

The cooling of the drying plates is switched off after which these plates are slowly raised in temperature. The drying period takes about 24 hours. The thermostat for the plate heating is switched on after the product-temperature has been raised above 10 °C and has become equal to the plate temperature and after ice in the flasks can no longer be seen. The temperature of the thermostat is adjusted to 30 °C and drying is carried out during about 5 hours. The thermostat, vacuum pump and the cooling of the condensor are switched off and the vacuum of the drying room is eliminated by supplying of nitrogen filtered through a 0.2 μm filter. The flasks are closed in the freeze drying equipment. After removal from the freeze drying equipment the flasks are sealed with aluminium folding capsules by means of Fermpress handcrimper type H-207.

The so obtained charges are subsequently analyzed with regard to the content of sodium amoxicillin, the content of decomposition products, the water content, the content of sodium chloride, clearness of the solution, pH of the solution, infra red spectrum and PMR spectrum.

Analyzing results of the prepared batch were the following :

| | |
|---|---|
| content (microbiological) of sodium amoxicillin | 93.3 % |
| content (mercurimetrical) of sodium amoxicillin | 94.4 % |
| content of decomposition products (mercurimetrical) | 3.6 % |
| solubility : 10 g/v % solution in water remains clear for at least 1 hour | |
| pH of freshly prepared solution in water 10 g/v % | 8.7 % |
| water content (Karl Fischer) | 2.2 % |

The structure of sodium amoxicillin was confirmed by IR and PMR spectra.

## Example 2

Using processes similar to that of Example 1, several charges of the sodium amoxicillin compositions are prepared whereby a number of parameters are varied as to the corresponding ones in Example 1.

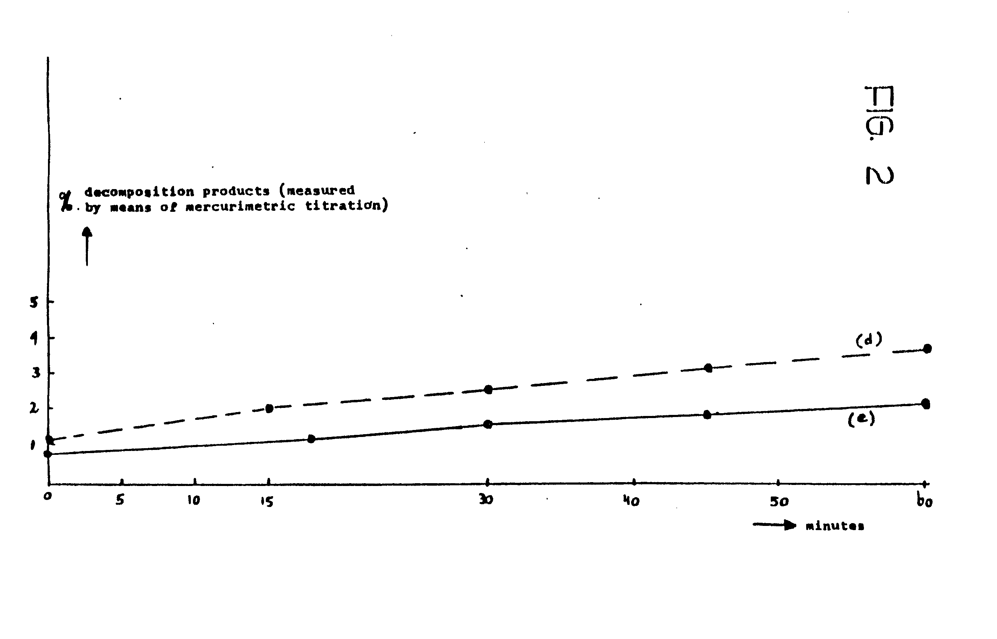

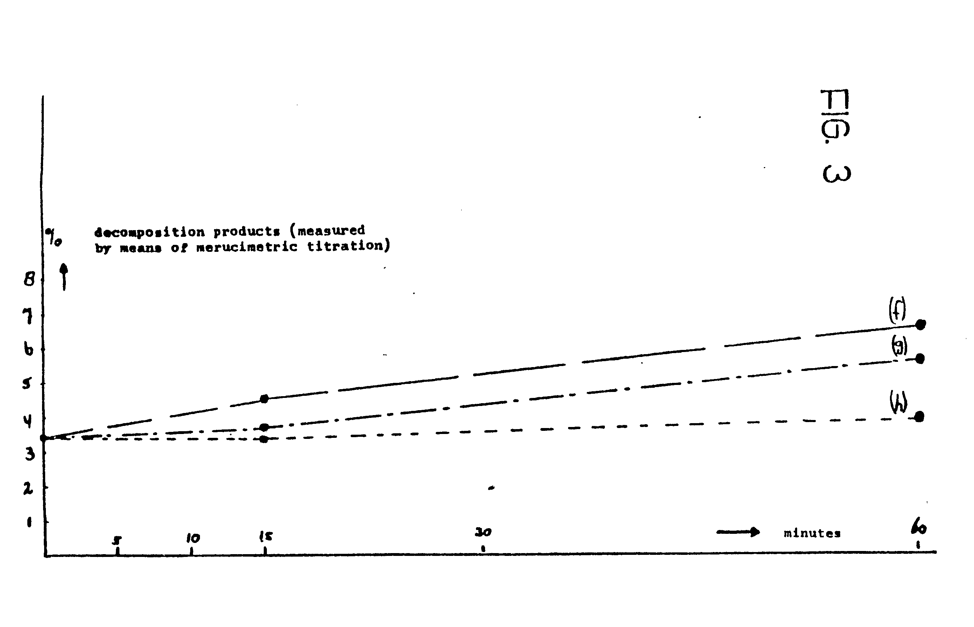

The results obtained according to Example 1 and 2 are summarized in the following table 1 and in the figures 2 and 3.

(See Table page 6)

In figure 2 the relationship between the stability of a 5 % and 10 % by weight solution of sodium amoxicillin, before the freezing and freeze drying, and time has been expressed, whereby the curve (d) relates to a 10 % by weight solution of sodium amoxicillin and curve (e) relates to 5 % by weight solution. Along the axis the percentage of decomposition products, measured by means of mercurimetric titration, and the time are plotted.

In figure 3 the relationship between the stability of the freeze dried sodium amoxicillin dissolved in water and time has been expressed, whereby the curve (f) relates to a 20 % by weight solution, curve (g) relates to a 10 % by weight solution and curve (h) relates to a 5 % by weight solution.

Along the axis the percentage of decomposition products measured by mercurimetric titration, and the time are plotted.

The freeze dried starting preparation as used for figure 3 was prepared by suspending amoxicillin trihydrate in water at room temperature, followed by the addition of a 15 mol% excess of sodium hydroxide, neutralization with a hydrochloric acid solution to a 5 % mol excess of sodium hydroxide, filtration of the obtained solution by means of a 0.2 μ filter and filling into F 20 flasks 10 ml of a 5 % by weight solution of sodium amoxicillin followed by freezing and freeze drying.

The flasks containing the freeze dried preparation were stored at 5 °C for 10 weeks.

## Example 3

2.58 mol (1.082 g) of amoxicillin trihydrate are suspended at 20-25 °C within 10 minutes by means of a Vibro Mischer in about 12.5 l of pyrogen free water. 2.97 mol (119 g) of sodium hydroxide in 2 l of pyrogen free water are added with a rate of about 380 ml/min under vigorously stirring. The clear solution is immediately thereafter neutralized with 0.26 mol (516 ml 0.50 N) of hydrochloric acid. The solution is adjusted with pyrogen free water to 20.0 l (20.28 kg) and mixed. The solution is filtered under aseptical conditions by means of a Seitz filter, having 9 EKS plate of 20 × 20 cm. The filtrate is collected under aseptical conditions in 4 l flasks. The solution is transmitted under aseptical conditions on sterilized

## Analysis results of testcharges (after freeze drying)

| charge amount | temp. of pre-paration of solution °C | excess of NaOH before neutra-lization mol % | excess NaOH after neutra-lization mol % | sodium amoxicil-lin(mer-curometri-cally de-termined) % | sodium amoxi-cillin theore-thical % | decomp. products (mercuri metrically deter-mined) % | water con-tent % | 10 g/v % solution clearness | measured pH |
|---|---|---|---|---|---|---|---|---|---|
| A 20 flasks à 0,5 g | 20—25 | 15 | 5 | 94.3 | 95,8 | 3,6 | 2,2 | clear | 8,7 |
| D 10 flasks à 1 g | 20—25 | 15 | 5 | — | — | — | — | turbid | 8,75 |
| G 50 flasks à 0,5 g | 20—25 | 15 | 5 | 95.8 | 95,8 | 3,3 | 2,3 | clear | 8,7 |

The characterizing amoxicillin structure of all tested charges could be confirmed by IR and PMR spectra.

plastic foil which is secured on the drying plates and temperature sensors are fixed and the drying room is closed. The foil has previously been secured on the drying plates and has an upstanding rim of a height of 4 cm supported by a sterilized metal frame work, while the foil has been secured in such a way, that an optimal contact is obtained of the solution with the drying plates. The drying plates of the freeze drying equipment are preferably previously cooled to a temperature of — 40 °C. The cooling capacity of the freeze dryer has to be selected large enough in order to freeze in the solution, having a level of about 1 cm within 60 minutes to — 10 °C. The time between the suspension of the amoxicillin trihydrate and the closing of the freeze drying equipment after pouring out the solution on the plates does not exceed 30 minutes. As soon as a formation of ice has occured and the temperature of the product is lowered to about — 10 °C, the condensor is switched on and the pressure in the drying room is lowered to 1 Torr = 133 N/m². After the pressure in the drying room has been kept for 1 hour at 1 Torr the cooling of the plates is switched off. Heating of the drying plates is switched on whereby the temperature of the heating equipment is slowly and linearly raising to 25 °C during 60 minutes. During the drying period which takes about 35 hours, the temperature of the product, the heater of the plates, the drying plates themselves, and the condensor and the pressure in the drying room and in the condensor are recorded. By measuring the pressure differences between the drying room and condensor made during short turning off of the connection between drying room and condensor there is determined when the product is practically dry. The secondary drying by means of a diffusion pump is started and takes 6 hours. At the same time the heating of the drying plate is adjusted to 30 °C. The vacuo of the drying room is eliminated by sypplying dry nitrogen, filtered through a sterilized 0.2 μm filter. The dried sodium amoxicillin is removed from the drying room and stored under nitrogen at 5 °C under aseptical conditions.

According to the foregoing process three additional charges of sodium amoxicillin are freeze dried. The four charges of 1 kg are ground by means of a milling equipment such as a Peppink mill under aseptical conditions until a sieve of 2 mm can be passed. The charges are combined and quantities of 250, 500 or 1 000 mg are brought into sterilized injection flasks by means of e. g. Hofflicher Karg filling equipment under dry and aseptical conditions. Under the same conditions the flasks are provided with sterilized rubber stoppers and sealed with aluminium folding capsules. The analysis results of the prepared combined charge are as follows :

| | |
|---|---|
| content (microbiological) of sodium amoxicillin | 931 μg/mg |
| content (mercurimetrical) of sodium amoxicillin | 94,1 % |
| content (mercurimetrical) of decomposition products | 3,7 % |
| solubility : 10 g/v % solution in water remains clear for at least 1 hour, | |
| pH of a freshly prepared solution in water 10 g/v % | 8,8 |
| water content (Karl Fischer) | 2,9 % |

## Example 4

A batch of 1 kg of freeze dried sodium amoxicillin is prepared according to a process similar to that described in example 4, but which essentially differs from that process in that after the addition of a solution of 118.7 g sodium hydroxide in 2 litres of pyrogen free water at a rate of 350 to 400 ml/minute, a part of the sodium hydroxide is immediately neutralized by the addition of 500 ml of a 0.50 N hydrochloric acid solution in about one minute under vigorous stirring, giving rise to a final excess of sodium hydroxide of 5.3 mol%, and whereafter the clear solution is adjusted to a total weight of 20.3 kg with pyrogen frée water.

The analysis results of the obtained batch were as follows :

| | |
|---|---|
| content (mercurimetrical) of decomposition products | |
| solubility : 10 g/v % solution in water remains clear for at least one hour, | |
| while the freshly prepared solution is as clear as water | 4.3 % |
| colour of freshly prepared solution in water 10 g/v % | < Y 5 |
| pH of freshly prepared solution in water 10 g/v % | 8.75 |
| colour of freshly prepared solution in water 20 g/v % | < Y 5 |
| pH of freshly prepared solution in water 20 g/v % | 8.78 |
| water content (Karl Fischer) | 3.85 % |

## Example 5

According to exactly the same process as described in example 4, a batch of 1 kg of sodium amoxicillin was prepared, the analysis results of which were as follows :

| | |
|---|---|
| content (mercurimetrical) of decomposition products | 3.5 % |
| solubility : 10 g/v % solution in water remains clear for a least one hour, | |
| while the freshly prepared solution is as clear as water | |
| colour of freshly prepared solution in water 10 g/v % | < Y 6 |

| | |
|---|---|
| pH of freshly prepared solution in water 10 g/v % | 8.30 |
| colour of freshly prepared solution in water 20 g/v % | ⩽ Y 6 |
| pH of freshly prepared solution in water 20 g/v % | 8.80 |
| water content (Karl Fischer) | 3.3 % |

## Claims

1. Process for the preparation of dry sodium amoxicillin preparations which may easily be converted into suitable injection solutions, whereby amoxicillin trihydrate is dissolved by adding an excess of sodium hydroxide and a solution with a final excess of sodium hydroxide from 3 to 5 mol% in relation to the amoxicillin is produced and said solution is freeze dried, characterized in that an excess of sodium hydroxide from 9 to 15 mol% over that required to form sodium amoxicillin is added gradually, but as fast as possible and with thorough mixing to an aqueous suspension of amoxicillin trihydrate until the amoxicillin has completely dissolved, and then a part of the excess sodium hydroxide is immediately neutralized with hydrochloric acid to said final excess at a temperature between 0 and 30 °C.

2. Process according to claim 1, characterized in that the amounts of amoxicillin trihydrate, sodium hydroxide and hydrochloric acid are selected in such a way that the concentration of sodium amoxicillin in the solution to be frozen is not more than 5 % by weight.

3. Process according to claim 1 or 2, characterized in that the steps from the preparation of the solution up to and including the freezing are completed within 90 minutes.

## Patentansprüche

1. Verfahren zur Herstellung von trockenen Natrium-amoxicillin-Zubereitungen, die leicht in geeignete Injektionslösungen übergeführt werden können, wobei Amoxicillin-trihydrat durch Zusatz eines Überschusses von Natriumhydroxyd gelöst und eine Lösung mit einem Endüberschuß von 3 bis 5 mol-% Natriumhydroxyd im Verhältnis zu dem Amoxicillin gebildet und diese Lösung gefriergetrocknet wird, dadurch gekennzeichnet, daß ein Überschuß von 9 bis 15 Mol-% Natrium-hydroxyd über die zur Bildung von Natrium-amoxicillin erforderliche Menge allmählich, aber so schnell wie möglich und unter gründlichem Vermischen zu einer wässerigen Suspension von Amoxicillin-trihydrat zugesetzt wird, bis das Amoxicillin völlig gelöst ist, worauf ein Teil des überschüssigen Natriumhydroxyds sofort mit Chlorwasserstoffsäure bis zu dem erwähnten Endüberschuß bei einer Temperatur von 0 bis 30 °C neutralisiert wird.

2. Verfahren nach Anspruch 1, dadurch gekennzeichnet, daß die Mengen an Amoxicillin-trihydrat, Natrium-hydroxyd und Chlorwasserstoffsäure so gewählt werden, daß die Konzentration von Natrium-amoxicillin in der zu gefrierenden Lösung nicht mehr als 5 Gew.-% beträgt.

3. Verfahren nach Anspruch 1 oder 2, dadurch gekennzeichnet, daß die Verfahrensschritte von der Herstellung der Lösung bis zum Gefrieren und einschließlich des Gefrierens innerhalb von 90 min durchgeführt werden.

## Revendications

1. Procédé de fabrication de préparations d'amoxicilline de soude sèches qui peuvent aisément être converties en des solutions d'injection appropriées, suivant lequel du trihydrate d'amoxicilline est dissous par addition d'un excès d'hydroxyde de soude et une solution ayant un excès final d'hydroxyde de soude de 3 à 5 % molaires par rapport à l'amoxicilline est formée et cette solution est lyophilisée, caractérisé en ce qu'une quantité d'hydroxyde de soude excédant de 9 à 15 % molaires celle nécessaire pour former de l'amoxicilline de soude est ajoutée graduellement, mais aussi vite que possible et en agitant bien, à une suspension aqueuse de trihydrate d'amoxicilline jusqu'à ce que l'amoxicilline se soit complètement dissoute, et ensuite une partie de l'excédent d'hydroxyde de soude est immédiatement neutralisée avec de l'acide hydrochlorique jusqu'à cet excès final à une température entre 0 et 30 °C.

2. Procédé selon la revendication 1, caractérisé en ce que les quantités de trihydrate d'amoxicilline, d'hydroxyde de soude et d'acide hydrochlorique sont choisies de telle façon que la concentration d'amoxicilline de soude dans la solution destinée à être congelée ne dépasse pas 5 % en poids.

3. Procédé selon la revendication 1 ou 2, caractérisé en ce que les étapes du procédé de la préparation de la solution jusqu'à et y compris celle de la congélation sont complétées en moins de 90 minutes.

FIG.1

0 009 845

bours

1

0 009 845

FIG. 2

% decomposition products (measured by means of mercurimetric titration)

(d)

(e)

minutes

FIG. 3